# EUROPEAN PATENT APPLICATION

(11) **EP 4 840 310 A1**
(43) Date of publication of application: **18.11.2026**
(21) Application number: 25176796.8
(22) Date of filing: 15.05.2025
(51) Int. Cl.: A01N 3/00, A01N 37/44, A01N 63/20, C12N 1/20, A01P 15/00, A01P 21/00

(54) **ETHYLENE SYNTHESIS INHIBITOR AND METHOD OF USING THE SAME TO REGULATE FLOWERING OF ORCHID**

(71) Applicant: National Chung-Hsing University, Taichung City 402 (TW)
(72) Inventor: Hung, Shih-Hsun Walter, 402 Taichung City (TW); Huang, Chieh-Chen, 402 Taichung City (TW)
(74) Representative: Lang, Christian

(57) **Abstract**

An ethylene synthesis inhibitor and a method of using the same to regulate the flowering of an orchid are provided. The ethylene synthesis inhibitor contains an effective amount of an endophyte and/or its metabolite and can inhibit the expression of genes associated with ethylene synthesis in orchids. Therefore, administering an effective amount of the ethylene synthesis inhibitor to an orchid can inhibit the biosynthesis of ethylene in the orchid to shorten the time required for flower induction, to increase the duration of full bloom, and to delay flower withering.

## Description

### BACKGROUND OF THE INVENTION

### 1. Technical Field

The present invention relates to a composition, and a method thereof, for regulating the flowering period of a plant. More particularly, the invention relates to an ethylene synthesis inhibitor and a method of using the same to regulate the flowering of a plant in the family *Orchidaceae.*

### 2. Description of Related Art

Commonly known as orchids, plants in the family *Orchidaceae* constitute the largest family of angiosperms and are one of the most important potted flowering plants on the global flower market. For example, orchids are second only to poinsettia (*Euphorbia pulcherrima*) in the United States in terms of sales and are the best-selling potted flowering plants in Japan and the Netherlands. In other words, orchids are plants of high economic value. *Phalaenopsis* orchids are of the genus *Phalaenopsis* in the family *Orchidaceae* and are highly valued ornamental plants, growing well in a ventilated environment where the temperature is 20-25°C and where there is diffused light.

Currently, the worldwide standard method of *Phalaenopsis* orchid reproduction is to perform meristem culture, and after transplantation, an orchid plantlet thus produced must go through a young plant period before reaching adulthood, i.e., before having the ability to bloom; in other words, the plantlet will not produce flowers until in its adulthood. The young plant periods of orchids vary from one species to another and average about two to three years, meaning it takes a rather long time for an orchid plantlet to grow into a state where it can bloom. To increase the economic value of orchids, it is important to control the time points at which they flower. The most common method of flower induction nowadays is to exercise temperature control, i.e., to use a low temperature to induce the production of flower buds from a mature plant and a temperature ranging from 20 to 25°C to increase the length of the/each spike and the percentage of flowering. While orchids can be induced to flower by temperature control, only mature orchids can be so induced, and the same temperature control cannot extend the flowering periods.

### BRIEF SUMMARY OF THE INVENTION

The primary objective of the present invention is to provide an ethylene synthesis inhibitor and a method of using the same to regulate the flowering of a plant in the family *Orchidaceae.* It is desirable that the flower induction period of a plant in the family *Orchidaceae* can be shortened by adding to the plant a non-chemically synthesized composition that has the activity to inhibit ethylene synthesis, in order to boost the competitiveness of the orchid industry.

Another objective of the present invention is to provide an ethylene synthesis inhibitor and a method of using the same to regulate the flowering of a plant in the family *Orchidaceae,* wherein the ethylene synthesis inhibitor can effectively extend the flowering periods of plants in the family *Orchidaceae* to increase the economic value of such plants.

To achieve the foregoing objectives, the present invention discloses an ethylene synthesis inhibitor that essentially includes *Burkholderia seminalis* strain 869T2 or its metabolite and that can be used to regulate the expression of the flowering and ethylene synthesis-related genes in a plant in the family *Orchidaceae.*

*Burkholderia seminalis* strain 869T2 was deposited with the Food Industry Research and Development Institute, Taiwan (FRIDI) on March 18, 2024 under the accession number BCRC911214 and also deposited with Deutsche Sammlung von Mikroorganismen and Zellkulturen GmbH, Inhoffenstrasse 7B, D-38124 Braunschweig, Germany (DSMZ) on May 5, 2025.

The flowering and ethylene synthesis-related genes include *ACS7, ACS3, FT1 (FLOWERING LOCUS T1*)*, TFL1 (TERMINAL FLOWER1*), *LFY* (*LEAFY*)*, EDF, FYF2,* and*IAA1.*

The metabolite of *Burkholderia seminalis* strain 869T2 contains rhizobitoxin, which can be used to inhibit endogenous ethylene synthesis and ethylene signaling in plants.

As the ethylene synthesis inhibitor has the activity to inhibit ethylene biosynthesis and ethylene signaling in plants and to encourage and increase the expression of flowering-related genes, another embodiment of the present invention discloses a method of regulating the flowering of a plant in the family *Orchidaceae.* The method involves administering the ethylene synthesis inhibitor disclosed herein to a plant in the family *Orchidaceae* or to a cultivation medium of the plant in order to shorten the flower induction period, and extend the flowering period, of the plant.

The ethylene synthesis inhibitor is administered while the plant in the family *Orchidaceae* is in a young plant period.

The ethylene synthesis inhibitor is administered at least once, preferably at least three times. When the ethylene synthesis inhibitor is administered for at least three times, it is administered at least once in each of stages 1 to 3 of its young plant period.

### BRIEF DESCRIPTION OF THE SEVERAL VIEWS OF THE DRAWINGS

FIG. 1 shows endophytic colonization of strain 869T2 that has been inoculated into the root and leaf tissue fluids of *Phalaenopsis* Taisuco Anna (K71303) for different numbers of days.
FIG. 2 shows the bacterial counts of strain 869T2 that has been inoculated into the root and leaf tissue fluids of *Phalaenopsis* orchid species A for two weeks.
FIG. 3 shows the bacterial counts of strain 869T2 that has been inoculated into the root and leaf tissue fluids of *Phalaenopsis* orchid species B for two weeks.
FIG. 4 shows the bacterial counts of strain 869T2 that has been inoculated into the root and leaf tissue fluids of *Phalaenopsis* orchid species C for two weeks.
FIG. 5 shows the bacterial counts of strain 869T2 that has been inoculated into the root and leaf tissue fluids of *Phalaenopsis* orchid species D for two weeks.
FIG. 6 shows the flowering states of the CK group and the 869T2 group of *Phalaenopsis* Taisuco Anna 316 days after the first inoculation (316 DAI), with CK representing the control group, and 869T2 representing the strain 869T2 inoculation group.
FIG. 7 shows the flowering states of the CK group and the 869T2 group of *Phalaenopsis* Taisuco Anna 447 days after the first inoculation (447 DAI), with CK representing the control group, and 869T2 representing the strain 869T2 inoculation group.
FIG. 8 shows the percentages of flowering of the CK group and the 869T2 group of *Phalaenopsis* Taisuco Anna after cultivation for different number of days, with CK representing the control group, and 869T2 representing the strain 869T2 inoculation group.
FIG. 9 shows the numbers of flowers of the CK group and the 869T2 group of *Phalaenopsis* Taisuco Anna after cultivation for different number of days, with CK representing the control group, and 869T2 representing the strain 869T2 inoculation group.
FIG. 10 shows an analysis result of the anthocyanin contents of the buds collected from the CK group and the 869T2 group of *Phalaenopsis* Taisuco Anna 459 days after the first inoculation (459 DAI), with CK representing the control group, and 869T2 representing the strain 869T2 inoculation group.
FIG. 11 shows an analysis result of the total protein contents of the buds collected from the CK group and the 869T2 group of *Phalaenopsis* Taisuco Anna 459 days after the first inoculation (459 DAI), with CK representing the control group, and 869T2 representing the strain 869T2 inoculation group.
FIG. 12 shows an analysis result of the acetaldehyde contents and ethanol contents of the buds collected from the CK group and the 869T2 group of *Phalaenopsis* Taisuco Anna 459 days after the first inoculation (459 DAI), with CK representing the control group, and 869T2 representing the strain 869T2 inoculation group.
FIG. 13 shows an analysis result of the glucose contents of the buds collected from the CK group and the 869T2 group of *Phalaenopsis* Taisuco Anna 459 days after the first inoculation (459 DAI), with CK representing the control group, and 869T2 representing the strain 869T2 inoculation group.
FIG. 14 shows an analysis result of the gluconic acid contents of the buds collected from the CK group and the 869T2 group of *Phalaenopsis* Taisuco Anna 459 days after the first inoculation (459 DAI), with CK representing the control group, and 869T2 representing the strain 869T2 inoculation group.
FIG. 15 shows an analysis result of the lactic acid contents of the buds collected from the CK group and the 869T2 group of *Phalaenopsis* Taisuco Anna 459 days after the first inoculation (459 DAI), with CK representing the control group, and 869T2 representing the strain 869T2 inoculation group.
FIG. 16 shows the relative expression levels of flowering regulation mechanism-related genes of *Phalaenopsis* orchids of the CK groups and of the 869T2 groups.
FIG. 17A shows an analysis result of the expressions of the mitochondrial *ATPase* gene in the leaves and roots of the CK group and the 869T2 group of *Phalaenopsis* Taisuco Anna, with CK representing the control group, and 869T2 representing the strain 869T2 inoculation group.
FIG. 17B shows an analysis result of the expressions of the chloroplast *ATPase* gene in the leaves and roots of the CK group and the 869T2 group of *Phalaenopsis* Taisuco Anna, with CK representing the control group, and 869T2 representing the strain 869T2 inoculation group.
FIG. 17C shows an analysis result of the expressions of the *RBCS1A* gene in the leaves and roots of the CK group and the 869T2 group of *Phalaenopsis* Taisuco Anna.
FIG. 17D shows an analysis result of the expressions of the *ALDH* gene in the leaves and roots of the CK group and the 869T2 group of *Phalaenopsis* Taisuco Anna.
FIG. 17E shows an analysis result of the expressions of the *ACS3* gene in the leaves and roots of the CK group and the 869T2 group of *Phalaenopsis* Taisuco Anna.
FIG. 17F shows an analysis result of the expressions of the *ACS7* gene in the leaves and roots of the CK group and the 869T2 group of *Phalaenopsis* Taisuco Anna.
FIG. 17G shows an analysis result of the expressions of the *IAA1* gene in the leaves and roots of the CK group and the 869T2 group of *Phalaenopsis* Taisuco Anna.
FIG. 17H shows an analysis result of the expressions of the *ABCB1* gene in the leaves and roots of the CK group and the 869T2 group of *Phalaenopsis* Taisuco Anna.
FIG. 17I shows an analysis result of the expressions of the *ABCG2* gene in the leaves and roots of the CK group and the 869T2 group of *Phalaenopsis* Taisuco Anna.
FIG. 17J shows an analysis result of the expressions of the *PDR5* gene in the leaves and roots of the CK group and the 869T2 group of *Phalaenopsis* Taisuco Anna.
FIG. 17K shows an analysis result of the expressions of the *FYF2* gene in the leaves and roots of the CK group and the 869T2 group of *Phalaenopsis* Taisuco Anna.
FIG. 17L shows an analysis result of the expressions of the *FYF2* gene in the buds, spike tips, and complete flowers of the CK group and the 869T2 group of *Phalaenopsis* Taisuco Anna.
FIG. 17M shows an analysis result of the expressions of the *EDF* gene in the leaves and roots of the CK group and the 869T2 group of *Phalaenopsis* Taisuco Anna.
FIG. 17N shows an analysis result of the expressions of the *EDF* gene in the buds, spike tips, and complete flowers of the CK group and the 869T2 group of *Phalaenopsis* Taisuco Anna.

### DETAILED DESCRIPTION OF THE INVENTION

The present invention discloses an ethylene synthesis inhibitor and a method of using the same to regulate the flowering of a plant in the family *Orchidaceae.* The ethylene synthesis inhibitor disclosed by the invention can inhibit the expression of the ethylene synthesis-related genes in plants in the family *Orchidaceae.* Therefore, administering an effective amount of the ethylene synthesis inhibitor to a plant in the family *Orchidaceae* can inhibit the biosynthesis of ethylene in the plant to shorten the time required for flower induction, to accelerate the plant growth, to increase the duration of full flowering, and to postpone the flower senescence. The economic benefits of orchids in the genus *Phalaenopsis* can thus be enhanced.

In one embodiment of the present invention, the ethylene synthesis inhibitor is administered to the plant in the family *Orchidaceae* before the plant enters the flowering period, and the number of times of administration is at least one.

In another embodiment of the present invention, the ethylene synthesis inhibitor is administered to the plant in the family *Orchidaceae* while the plant is in a young plant period, and the preferred administration frequency is at least once in each of stages 1 to 3 of the young plant period.

In one embodiment of the present invention, the ethylene synthesis inhibitor can be administered directly to any part of the plant in the family *Orchidaceae* or to the medium in/with which the plant in the family *Orchidaceae* is cultivated.

In one embodiment of the present invention, the main active ingredient of the ethylene synthesis inhibitor is an endophyte and/or its metabolite, and the endophyte and/or the metabolite has the activity to regulate the flowering-related genes, and inhibit the ethylene synthesis-related genes, in a plant in the family *Orchidaceae.*

The endophyte is a microorganism in the genus *Burkholderia* and has the activity to attach to and colonize the root of a host plant, to inhibit the expression of the ethylene synthesis-related genes in the plant and of their substrates, to stimulate respiration and photosynthesis of the plant, to regulate the carbon flow direction and energy production in the plant, and so on. Therefore, the ethylene synthesis inhibitor of the present invention can be used to shorten the time required for a plant in the family *Orchidaceae* to enter adulthood from its young plant period.

More specifically, the strain of the endophyte disclosed in the present invention is *Burkholderia seminalis* strain 869T2, abbreviated as *B. seminalis* strain 869T2 or simply as strain 869T2. *Burkholderia seminalis* strain 869T2 was deposited with the Food Industry Research and Development Institute, Taiwan (FRIDI) on March 18, 2024 under the accession number BCRC911214 and also deposited with Deutsche Sammlung von Mikroorganismen and Zellkulturen GmbH, Inhoffenstrasse 7B, D-38124 Braunschweig, Germany (DSMZ) on May 5, 2025.

Strain 869T2 is capable of regulating the expression of genes associated with the flowering and ethylene synthesis of plants in the family *Orchidaceae,* including reducing the expressions of such genes as *ACS7, ACS3, FT1* (*FLOWERING LOCUS T1*), *TFL1* (*TERMINAL FLOWER1*), *LFY* (*LEAFY*)*, eEDF,* and *FYF2.* Therefore, strain 869T2 can inhibit the ethylene synthesis and signaling in plants in the family *Orchidaceae,* thereby extending the flowering periods and postponing the flower senescence.

In another embodiment of the present invention, the metabolite of strain 869T2 contains rhizobitoxin.

In one embodiment of the present invention, the ethylene synthesis inhibitor contains an effective amount of strain 869T2 and/or its metabolite and may be added with different ingredients that are acceptable in agriculture, depending on the method of use or the dosage form to be used.

In one embodiment of the present invention, the dose in which the ethylene synthesis inhibitor is administered to a plant in the family *Orchidaceae* varies with such parameters as the growing density, the growth environment, and the species of the plant, and if such variations of the dose or of the administered quantity can be obtained by a person of ordinary skill in the art according to general common knowledge, the variations shall fall within the scope of the present specification and the appended claims without being bound by the examples given in the present specification.

The term "endophyte" refers to a microorganism that lives within a host plant (including the intercellular spaces, xylem, and phloem of the plant), that is advantageous to the growth of the host, and that does not cause any disease. An endophyte can improve the growth of its host or increase the level of tolerance and resistance of the host via the metabolite produced by the endophyte during its growing process.

The term "strain 869T2" refers to an endophyte that is isolated from vetiver grass and that is a Gram-negative bacterium in the genus *Burkholderia.* Strain 869T2 is generally cultured with an LB plate culture medium under the culture conditions of 37°C and 120 rpm, wherein the LB plate culture medium is prepared from commercially available Luria-Bertani broth powder, tryptone, a yeast extract, sodium chloride, and agar.

The whole genome sequence of strain 869T2 has been verified using sequencing platforms (Illumina and ONT), and the sequencing results show that strain 869T2 has three circular chromosomes each having a length of 3561262 bp, 3012498 bp, or 1448576 bp, with the average GC content being 67.05%. A comparison with the data in the Burkholderiales database reveals that the genome of strain 869T2 has 687 complete BUSCOs (99.9%), 1fragmented BUSCO (0.1%), and no missing BUSCO (0.0%). As shown in Table 1 below, strain 869T2 includes six complete sets of rRNA genes, 67 tRNA genes, four ncRNAs, 7084 protein-coding genes, and 104 pseudogenes.

**Table 1. Genomic characteristics of strain 869T2**

| Characteristic | Chromosome 1 | Chromosome 2 | Chromosome 3 |
|---|---|---|---|
| Accession no. | CP072520.1 | CP072521.1 | CP072522.1 |
| Size (bp) | 3561262 | 3012498 | 1448576 |
| GC content (%) | 67.1 | 67.5 | 65.5 |
| Protein-coding genes | 3210 | 2655 | 1219 |
| Pseudogenes | 19 | 37 | 48 |
| rRNA (ribosomal RNA) genes | 12 | 3 | 3 |
| tRNA (transfer RNA) genes | 60 | 5 | 2 |
| Other non-coding RNA genes | 4 | 0 | 0 |

The term "plant in the family *Orchidaceae,"* or the common name "orchid," refers to a plant in the second largest family of flowering plants, i.e., *Orchidaceae.* Plants in the family *Orchidaceae* can be divided by the growth environment into saprophytic orchids, terrestrial orchids, semi-terrestrial orchids, and epiphytic orchids. Orchids in the genera *Phalaenopsis, Dendrobium,* and *Taeniophyllum,* among others, are epiphytic orchids.

The term *"Phalaenopsis* orchids" refers to orchids in the genus *Phalaenopsis,* and these orchids are also known as "moth orchids." *Phalaenopsis* orchids are currently the most diversified flowering plants and the most widely distributed plants in the family *Orchidaceae.* Taiwan has two major species of *Phalaenopsis* orchids, namely *Phalaenopsis aphrodite* subsp. Formosana (abbreviated as *P. aphrodite*) and *Phalaenopsis equestris* (abbreviated as *P. equestris*)*.*

Generally speaking, the growing/cultivation method of a *Phalaenopsis* orchid includes five stages, the first three of which constitute the young plant period, and the last two of which constitute the adulthood. The first stage involves cultivating a tissue-cultured orchid plantlet under the conditions of about 25-28°C and about 10-13 hours of illumination per day until the plantlet has two leaves and three roots. The second stage involves transferring the two-leaf three-root plantlet into a container containing sphagnum moss as the main cultivation medium, and allowing the plantlet to grow in a greenhouse having the illumination conditions corresponding to a natural day/night cycle and a temperature of about 30°C, until the plantlet has about four to six leaves, which is time to transfer the plantlet to a larger container to start the third stage. The third stage is vernalization and lasts for a month, and the transplantation that initiates the third stage is typically carried out 40 weeks after the transplantation initiating the second stage. The growing conditions of the third stage include a temperature of about 19-21°C and a 10/14-hour day/night cycle. The fourth stage and the fifth stage involve cultivation at a temperature of about 24-26°C.

The term "vegetative growth period" refers to a period of growth between germination and flowering. During the vegetative phase, plants are carrying out photosynthesis and accumulating resources that will be needed for flowering and reproduction.

The term "reproduction growth period" refers to the process in the flowering plant including flower initiation and flower expansion to produce of separate male and female gametophytes that produce gametes.

The term "flower induction" refers to the physiological process in the plant by which the shoot apical meristem becomes competent to develop flowers, it usually uses a low temperature to induce the production of flower buds from a mature plant, wherein in this invention, the low temperature is 20-25°C. It can be used to let t accelerate the plant from the vegetative growth period into the reproduction growth period to have flowers.

The term "metabolite of strain 869T2" refers to the substances produced by strain 869T2 during its growth, wherein the substances include antibacterial substances, cyclic dipeptides, plant hormones, and other regulatory substances, as detailed in Table 2 below.

**Table 2. Ingredients of the metabolite of strain 869T2**

| Classification | Ingredients |
|---|---|
| Substances associated with environmental adaptation | Isopyrrolnitrin |
| | Oxypyrrolintrin |
| | Fluorppyrrolintrin |
| | 3-chloro-4-(2-nitrophenyl)-1H-pyrrole |
| | Flavacid |
| | Indole-3-ethanol |
| | Karnamicin A3, D1 |
| | Aerugine |
| | N-salicyoyl-2-amionopropan-1-ol |
| | N-salicyoyl-2-amionopropan-1,3-ol |
| | Indoleacryloisonitrile, Antibiotics B371 |
| | 7-chloro-4a-hydroxy-8-methoxy-N-methyl-tetracycline |
| | Indole-3-carbaldehyde |
| | Indole-3-carboxylic acid |
| Substances that promote plant growth | Indole-3-acetic acid |
| | Indole-3-acetic acid methyl ester |
| | Chloroindole |
| | Phenylacetic acid |
| | Benzylcyanide |
| | Trans-zeatin |
| | Zeatin-riboside |
| | Zeatin-2'-deoxyriboside |
| | 1'-methyl-zeatin |
| | Gibbestain-B |
| | Rhizobitoxin |
| | Salicylic acid |
| | PCN (pyocyanine) |
| | PCH (pyochelin) |
| | Neopyochelin |
| | Proferrioxamines |
| | Pyrroloquinoline quinone (PQQ) |
| Cyclic dipeptides (CDPs) | cyclo(L-Pro, L-Val) |
| | cyclo(L-Tyr, D-4OH-Pro) |
| | cyclo(L-Tyr, L-4OH-Pro) |
| | cyclo(L-4OH-Pro, D-Leu) |
| | cyclo(L-Pro, L-Ile) |
| | cyclo(L-Pro, L-Met) |
| | cyclo(L-Pro, D-Val) |
| | cyclo(L-Pro, D-Ile) |
| | cyclo(Asp, Leu) |
| | cyclo(D-Phe, D-Pro) |
| | cyclo(D-Phe, L-Pro) |
| | cyclo(L-Trp, L-Pro) |
| | cyclo(L-Arg, D-Pro) |
| | cyclo(L-Phe, L-Pro) |
| | cyclo(L-Tyr, L-Pro) |
| | cyclo(D-Pro, L-Tyr) |
| | cyclo(L-Leu, L-Pro) |
| | cyclo(D-Leu, D-Pro) |
| | cyclo(Leu, Pro) |
| | cyclo(Gly, D-Pro) |
| | cyclo(Phe, dehydroAbu) |
| | cyclo(hexenyl, Gly) |
| | cyclo(D-Pro, L-Val) |
| | cyclo(L-Leu, Gly) |
| | cyclo(Ala, OH-Pro)(S,S,R) |
| | cyclo(Ala, OH-Pro)(S,R,R) |
| | cyclo(D-pipecoliny, L-Ile) |
| | cyclo(Ala, Trp)(3R, 6R) |
| | cyclo(D-Tyr, D-Pro) |
| | cyclo(N-ME-Tyr, dehydroAbu) |
| | cyclo(L-Ile, L-Pro, L-Leu, L-Pro) |

The term "administer" or "add" refers to a means of making strain 869T2 or its metabolite contact a plant in the family *Orchidaceae,* such as spraying, inoculation, or flood irrigation.

The term "DAI" (days after inoculation) refers to the number of days after inoculation of the endophyte for the first time, and the expression "number DAI" is used herein. For example, 4 DAI means four days after inoculation of the endophyte for the first time, and 447 DAI means 447 days after inoculation of the endophyte for the first time. In some examples of the present invention, and for ease of description, "number DAI" is used to indicate the number of days of cultivation of a control group, which, however, has not been inoculated with the endophyte, and in that case, DAI refers to the number of days after the day on which the experimental group in the same experiment was inoculated for the first time.

The genes referred to in the present invention are well known to the skilled person in the art and are disclosed in publicly available literature, as listed below:
Jang S, Choi S-C, Li H-Y, An G, Schmelzer E. 2015. Functional Characterization of Phalaenopsis aphrodite Flowering Genes PaFT1 and PaFD. PLOS ONE 10: e0134987.
Owens LD, Lieberman M, Kunishi A. 1971. Inhibition of ethylene production by rhizobitoxine. Plant Physiology 48: 1-4.
Yasuta T, Satoh S, Minamisawa K. 1999. New Assay for Rhizobitoxine Based on Inhibition of 1-Aminocyclopropane-1-Carboxylate Synthase. Applied and Environmental Microbiology 65: 849-852.
Chen W-H, Jiang Z-Y, Hsu H-F, Yang C-H. 2021. Silencing of FOREVER YOUNG FLOWER-Like Genes from Phalaenopsis Orchids Promotes Flower Senescence and Abscission. Plant and Cell Physiology 62: 111-124.

The technical features of the present invention and their effects are described in more detail below with reference to some experimental examples and the accompanying drawings.

In the following examples, the inoculation concentration of the endophyte reagent was OD600 = 1.0 unless otherwise specified. This inoculation concentration is an illustrative concentration intended to validate the effect of the invention but not to limit the scope of the present specification or the claims. In other words, adjustments that are made, within a reasonable range, to the inoculation concentration by a person of ordinary skill in the art according to general common knowledge, as well as variations of the inoculation concentration within a reasonable range of error, shall fall within the scope of the patent protection sought by the applicant.

In the following examples, the qRT-PCR primers are listed in the Table 3.

**Table 3: List of qRT-PCR primer pairs used in the examples of this invention**

| Gene Product | Orchidstra ID | Primer pairs | Sequence Number ID (5' to 3') |
|---|---|---|---|
| Phalaenopsis ACTIN | NA | PaACT_F | SEQ ID No.:1 |
| | | PaACT_R | SEQ ID No.:2 |
| FYF2 | NA | PaFYF2_F | SEQ ID No.:3 |
| | | PaFYF2_R | SEQ ID No.:4 |
| EDF1 | NA | PeEDF1_F | SEQ ID No.: 5 |
| | | PeEDF1_R | SEQ ID No.:6 |
| ATP synthase gamma chain, chloroplastic | PATC140837 | PaATPSc_2F | SEQ ID No.:7 |
| | | PaATPSc_2R | SEQ ID No.:8 |
| ATP synthase subunit beta, mitochondrial | PATC145359 | PaATPSm_2F | SEQ ID No.:9 |
| | | PaATPSm_2R | SEQ ID No.:10 |
| Auxin-responsive protein IAA1-like | PATC124553 | PaIAA1_1F | SEQ ID No.:11 |
| | | PaIAA1_1R | SEQ ID No.:12 |
| Ribulose bisphosphate carboxylase small chain A, chloroplastic-like | PATC155003 | PaRBCS1A_1F | SEQ ID No.:13 |
| | | PaRBCS1A_1R | SEQ ID No.:14 |
| Aldehyde dehydrogenase family 2 member C4-like isoform X1 | PATC091464 | PaALDH_1F | SEQ ID No.:15 |
| | | PaALDH_1R | SEQ ID No.:16 |
| 1-aminocyclopropane-1-carboxylate synthase 7-like | PATC125418 | PaACS7_1F | SEQ ID No.:17 |
| | | PaACS7_1R | SEQ ID No.:18 |
| 1-aminocyclopropane-1-carboxylate synthase 3-like | PATC022615 | PaACS3_1F | SEQ ID No.:19 |
| | | PaACS3_1R | SEQ ID No.:20 |
| ABC transporter B family member 1 | PATC124474 | PaABCB1_1F | SEQ ID No.:21 |
| | | PaABCB1_1R | SEQ ID No.:22 |
| ABC transporter G family member 25 | PATC150168 | PaABCG2_1F | SEQ ID No.:23 |
| | | PaABCG2_1R | SEQ ID No.:24 |
| ABC transporter G family member 44-like | PATC158026 | PaPDR5_1F | SEQ ID No.:25 |
| | | PaPDR5_1R | SEQ ID No.:26 |

For the purpose of patent application, strain 869T2 that was used in the following examples was deposited at the Food Industry Research and Development Institute on March 18, 2024, and was given the accession number BCRC911214. An the *Burkholderia seminalis* strain 869T2 was also deposited with Deutsche Sammlung von Mikroorganismen and Zellkulturen GmbH, Inhoffenstrasse 7B, D-38124 Braunschweig, Germany (DSMZ) on May 5, 2025.

### Example 1: Growing Phalaenopsis orchids

Tissue-cultured plantlets of *Phalaenopsis* Taisuco Anna 'Taisuco K71303' were purchased from Taiwan Sugar Corporation, Tainan, Taiwan, and were grown in the following stages:
Stage 1: This was the growing stage, in which the plantlets were cultivated at 26°C in an environment with a 13/11-hour day/night cycle until each plantlet had two leaves and three roots.
Stage 2: The *Phalaenopsis* orchid plantlets were transferred to a greenhouse in order to be domesticated. The plantlets were then transplanted into pots having a diameter of about 5 cm and containing sphagnum moss. The greenhouse had a natural day/night cycle and a temperature of about 30°C.
Stage 3: Orchid plantlets each having about four to six leaves were cultivated in pots each having a diameter of about 8 cm. The cultivation temperature was about 20±1°C, the illumination conditions were those corresponding to a 10/14-hour day/night cycle, and the cultivation time was about one month.
Stage 4 and stage 5: Orchids entering the flowering period were cultivated in an environment where the temperature was about 25±1°C and where the illumination conditions were those corresponding to a 12/12-hour day/night cycle.

### Example 2: Endophytic cultivation test (1) on Phalaenopsis orchids and strain 869T2

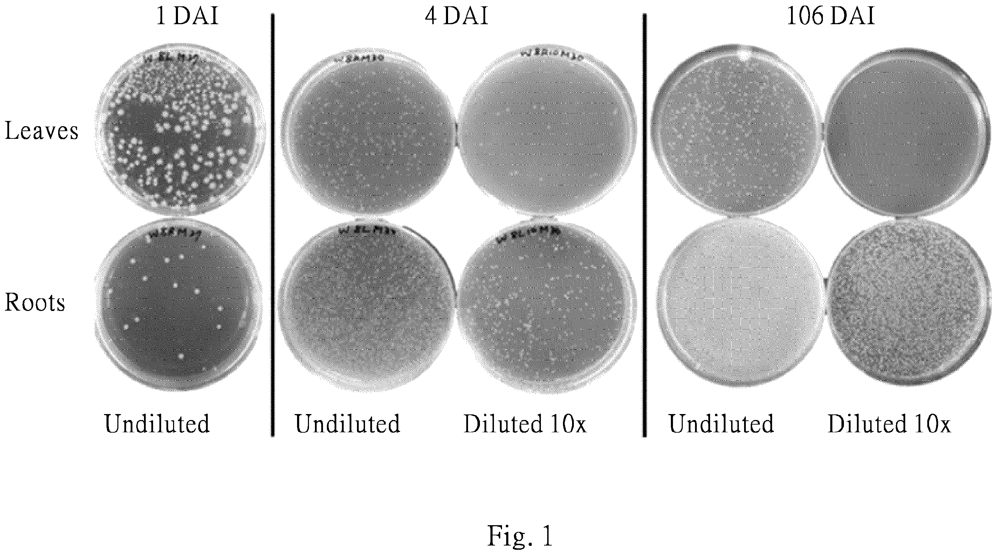

On the three days before stage 2 of growing *Phalaenopsis* orchids, the first inoculation of the endophyte reagent (i.e., *B. seminalis* 869T2) was performed. On the 21^{st} day and the 200^{th} day after the first inoculation, the second inoculation and the third inoculation were carried out respectively by flood irrigation into the sphagnum moss. On the 1^{st} day, the 4^{th} day, and the 106^{th} day after the first inoculation, the roots and leaves of the *Phalaenopsis* orchids were collected, subjected to surface sterilization (treatment with 0.6-1.5% NaOCl) for 5-20 minutes, and then washed with sterile double-distilled water. The treated roots and leaves of the *Phalaenopsis* orchids were then ground separately, and the resulting tissue liquids were collected, subjected to serial dilution (10×), laid on an LB agar plate, and cultured at 37°C for 24 hours. The results are shown in FIG. 1.

It can be known from the results in FIG. 1 that strain 869T2 was indeed capable of colonizing *Phalaenopsis* Taisuco Anna, and that the root was more suitable for colonization by strain 869T2 than the leaf as continual growth of strain 869T2 for at least 106 days after the first inoculation was observed in the root tissue fluid.

### Example 3: Endophytic cultivation test (2) on Phalaenopsis orchids and strain 869T2

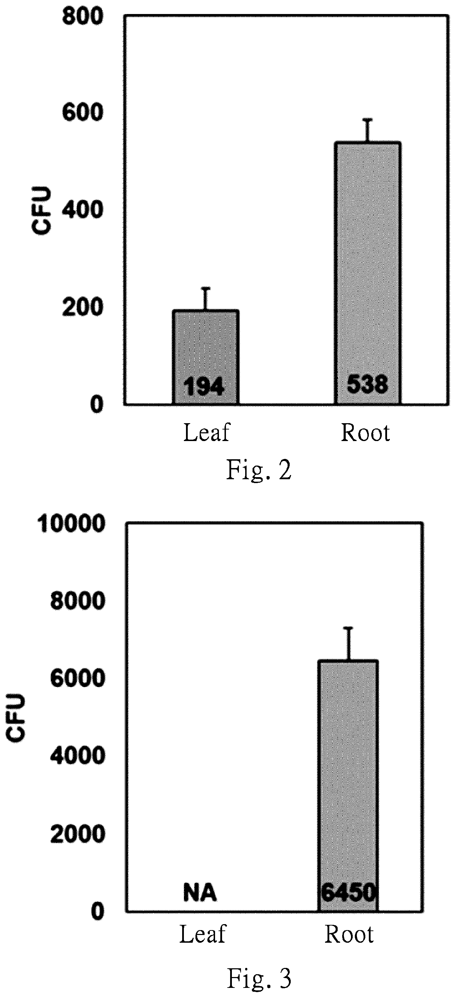

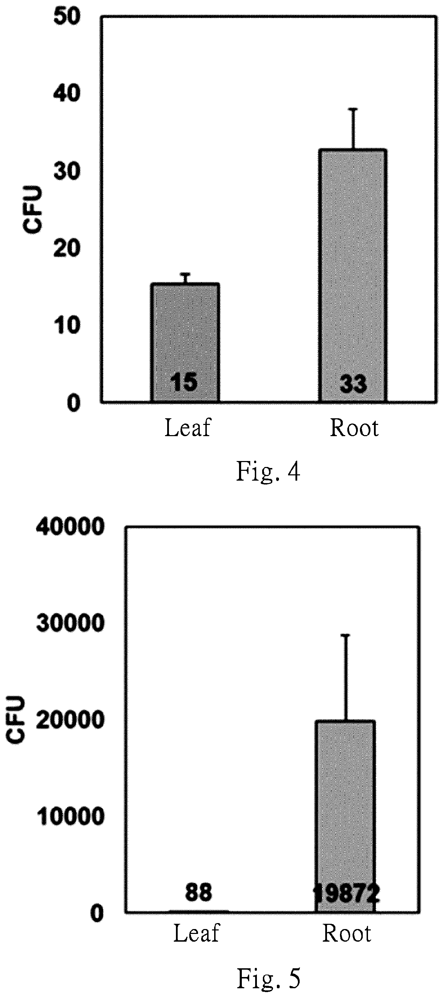

Using the process flow of example 2, strain 869T2 was inoculated separately into four other commercial species, namely species A to D, of *Phalaenopsis* orchids, and two weeks after the inoculation, the endophytic colonization of strain 869T2 in the root and leaf tissue fluids was examined by counting the number of fresh colony-forming units (CFUs) per gram, with the results shown in FIG. 2 to FIG. 5.

It can be known from the results in FIG. 2 to FIG. 5 that strain 869T2 was indeed capable of colonizing *Phalaenopsis* orchids of different species, and that the root was more suitable for endophytic colonization by strain 869T2 than the leaf.

### Example 4: Effect of strain 869T2 on the adulthood (flowering) of Phalaenopsis orchids

The tissue-cultured *Phalaenopsis* orchid plantlets were randomly divided into two groups: the control group (abbreviated as the CK group), which received no inoculation, and the strain 869T2 inoculation group (abbreviated as the 869T2 group), which received inoculation on the 1^{st} day, the 21^{st} day, and the 200^{th} day of cultivation, wherein except for the inoculation, the tissue-cultured *Phalaenopsis* orchid plantlets in the two groups were cultivated in the same way as in example 1.

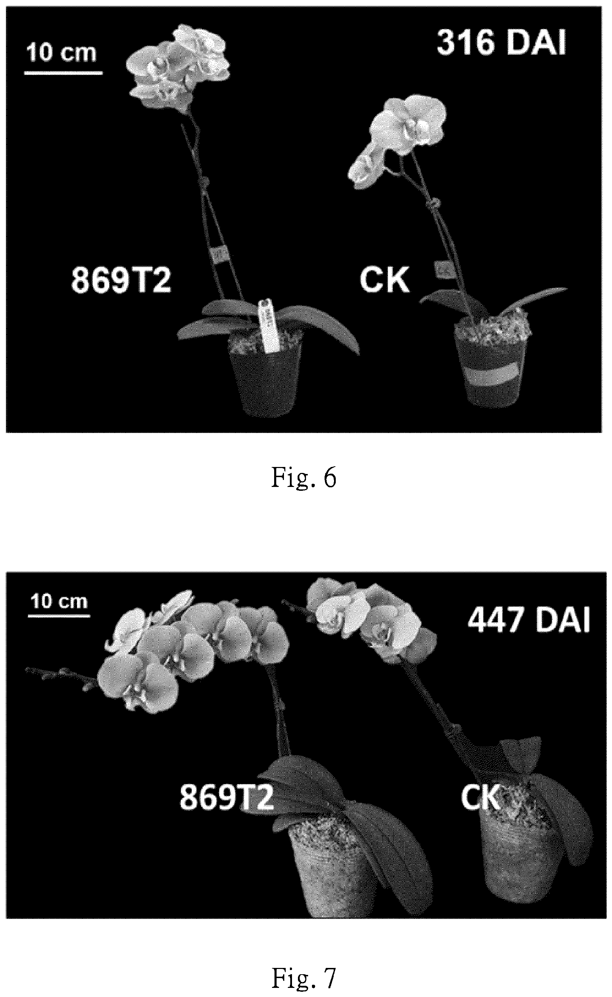

About 280 days into the cultivation, the *Phalaenopsis* orchids were subjected to flower induction for one month, and the effects of strain 869T2 on the flowering periods of the orchids were observed, as shown in FIG. 6 to FIG. 9.

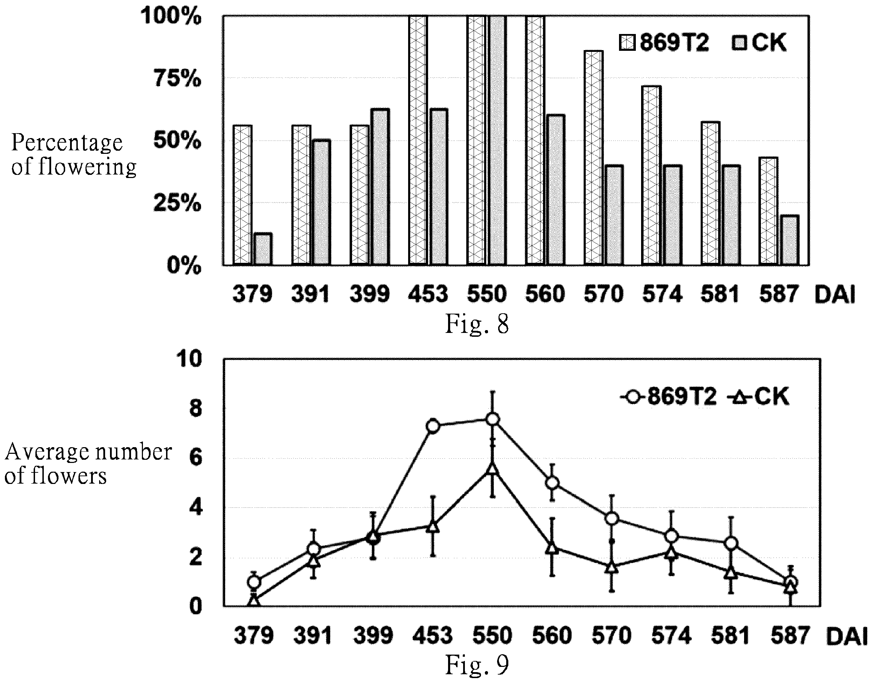

It can be known from the results in FIG. 6 and FIG. 7 that the *Phalaenopsis* orchid in the strain 869T2 inoculation group grew more exuberantly than that in the CK group. It can also be known from the results in FIG. 8 and FIG. 9 that the first bloom, as well as the existence of the inflorescence, of the *Phalaenopsis* orchid in the strain 869T2 inoculation group was about one week sooner than that of the CK group, and that the uniformity and quantity of the flowers of the 869T2 group were better than those of the CK group.

More specifically, full bloom of the CK group took place 550 DAI, with an average of five flowers per plant, and with more than 50% of the flowers of each plant completely withered 20 days after full bloom, leaving less than two flowers per plant. By contrast, the *Phalaenopsis* orchids in the 869T2 group came into full bloom 453 DAI, with a maximum of ten flowers and an average of seven flowers per plant, with the flowering periods lasting for about three months, and with an average of five flowers per plant 107 days after full bloom. The results in FIG. 8 and FIG. 9 show that the flowers of the *Phalaenopsis* orchids inoculated with strain 869T2 withered more slowly than those in the CK group (which was not inoculated with strain 869T2). More specifically, the withering of all the flowers of more than 50% of the plants in the 869T2 group did not occur until 587 DAI.

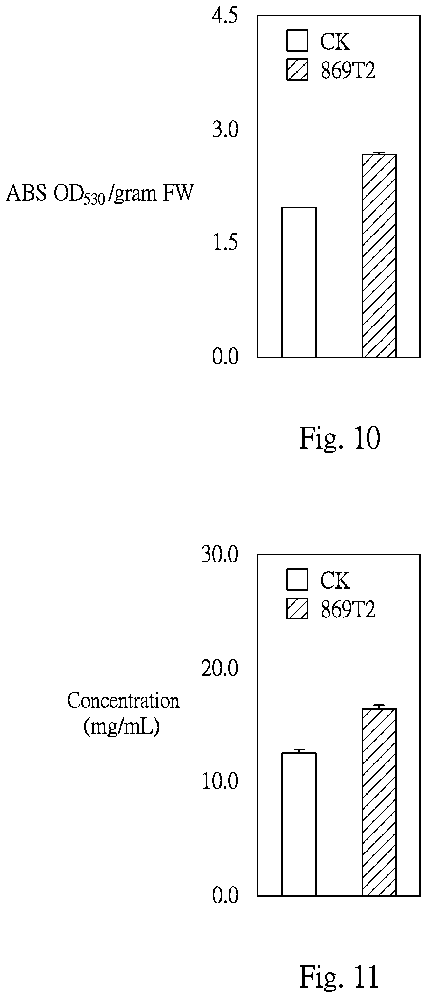

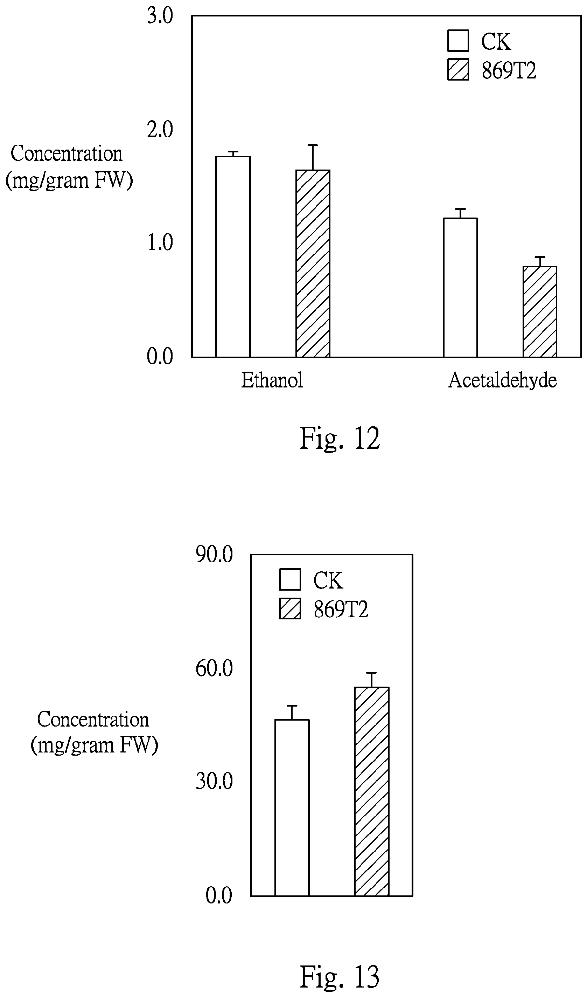

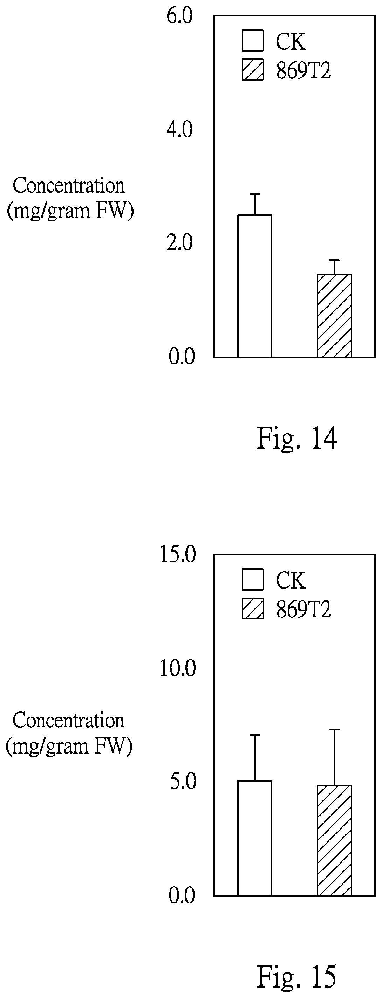

Furthermore, buds were collected from the CK group and the 869T2 group during the blooming periods (459 DAI) in order to have their total protein contents, anthocyanin contents, acetaldehyde contents, ethanol contents, gluconic acid contents, lactic acid contents, and glucose contents measured. The results are shown in FIG. 10 to FIG. 15.

It can be known from the results in FIG. 10 to FIG. 15 that the 869T2 group had a higher total protein content, anthocyanin content, and glucose content than the CK group, which was not inoculated with strain 869T2, and a lower acetaldehyde content, ethanol content, gluconic acid content, and lactic acid content than the CK group. It can be inferred from the results that the strain 869T2 disclosed in the present invention is an active substance capable of regulating the metabolic reactions in a plant in the family *Orchidaceae* in the flowering period and can advance the flowering time, extend the flowering period, and increase the number of flowers of a plant in the family *Orchidaceae* by raising the total protein content, anthocyanin content, and glucose content and lowering the acetaldehyde content, ethanol content, gluconic acid content, and lactic acid content in the plant.

### Example 5: Gene expression analysis (1)

Total RNAs were obtained from the roots and leaves of the CK group and the 869T2 group of the *Phalaenopsis* orchids during the flowering periods (410 DAI) and were sequenced by the Illumina Hi-Seq platform. It was found by subsequent analysis and identification that the leaf tissues of the 869T2 group and the CK group had 389 differential expression genes (hereinafter referred to as DEGs), including seven positive-regulation genes and 382 negative-regulation genes, and that the root tissues had 281 DEGs, including 127 positive-regulation genes and 154 negative-regulation genes.

The aforesaid DEGs were analyzed using the Kyoto Encyclopedia of Genes and Genomes (KEGG) database, and it was found that strain 869T2 is advantageous to such metabolic pathways in *Phalaenopsis* orchids as the MAPKs signaling pathways, the plant hormone signaling pathways, the ATPase pathways, and the carbon fixation pathways. More specifically, strain 869T2 can perform position regulation on the following genes: chloroplast or mitochondrial ATPase (EC 7.1.2.2) gene, ribulose 1,5-bisphosphate carboxylase (RuBisCo) (EC 4.1.1.39) gene, acetaldehyde dehydrogenase (ALDH) gene (EC 1.2.1.3), which is associated with glycolysis/gluconeogenesis, auxin/indole-3-acetic acid (Aux/IAA) gene, isocitrate dehydrogenase (IDH) (EC 1.1.1.42) gene, α-ketoglutarate dehydrogenase (α-KGDH) (EC 1.2.4.2) gene, succinyl-CoA synthetase (SCS) (EC 6.2.1.5) gene, succinate dehydrogenase (SDH) (EC 1.3.99.1) gene, and malate dehydrogenase (MDH) (EC 1.1.1.37) gene; and perform negative regulation on the following gene: 1-aminocyclopropane-1-carboxylic acid (ACC) synthase (EC 4.4.1.14) gene.

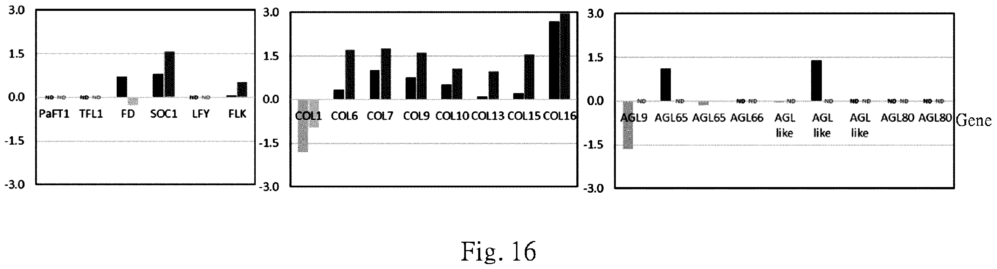

An analysis on how the flowering regulation mechanism-related genes of *Phalaenopsis* orchids were expressed in the roots and leaves of the 869T2 group and of the CK group was also conducted, and the results are shown in FIG. 16. It can be known from the results in FIG. 16 that: in relative terms with respect to the CK group, the expressions of *FT1 (FLOWERING LOCUS T1*)*, TFL1 (TERMINAL FLOWER1*), and *LFY* (*LEAFY*) in the 869T2 group were far lower than the preset threshold values; the expression of *FD* (*FLOWERING LOCUS D*)*,* which gene encodes an FT1-interacting protein, was increased in the leaves but reduced in the roots; and the expressions of *SOC1* (*SUPPRESSOR OF OVEREXPRESSION OF CO 1)* and *FLK* (*FLOWERING LOCUS KH DOMAIN*) genes were increased in both the leaves and the roots. In addition, a total of eight *CO* (*CONSTANS*) and *COL* (*CONSTANS-like*) genes were identified in the leaves and the roots, wherein except for *COL1,* all the genes showed an increase in expression in the *Phalaenopsis* orchids added with strain 869T2, and wherein nine genes that encode AGL (AGAMOUS-like), MADS-box, and AGL-like proteins were expressed in the leaves, with two of them showing an increase in expression in the leaves, three of them a decrease in expression in the leaves, and only one of them an increase in expression in the roots.

The strain 869T2 disclosed in the present invention can regulate the expression of the foregoing genes and can therefore be used to manipulate the carbon flow and energy in *Phalaenopsis* orchids, inhibit ethylene synthesis in *Phalaenopsis* orchids, and thereby delay flower withering of *Phalaenopsis* orchids. In other words, the strain 869T2 disclosed in the invention can function as an ethylene synthesis inhibitor or the active ingredient of a composition for extending the flowering period.

### Example 6: Gene expression analysis (2)

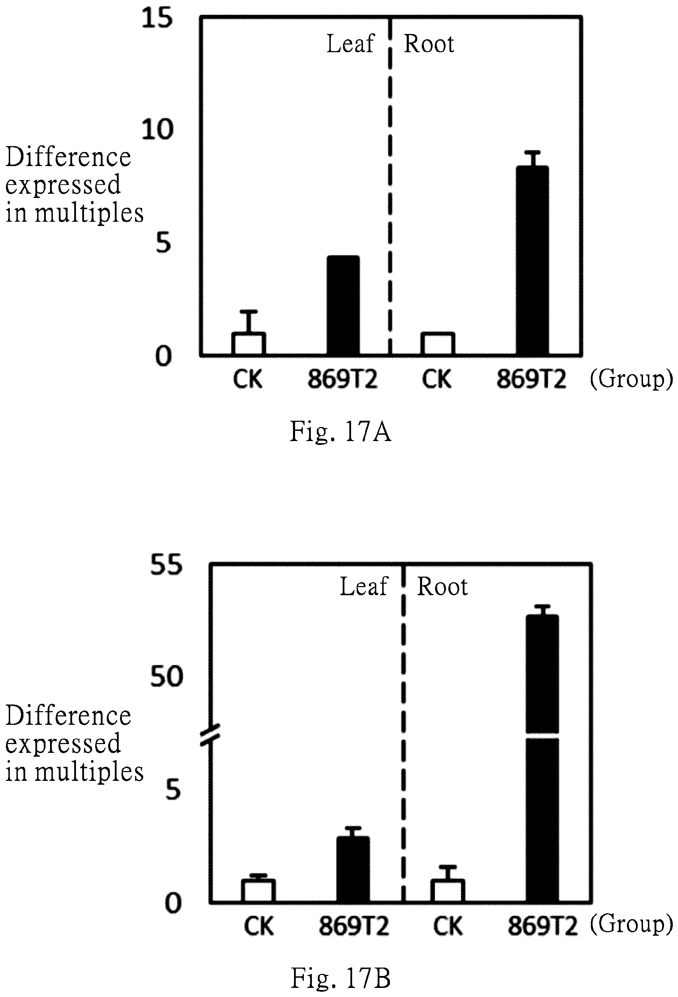

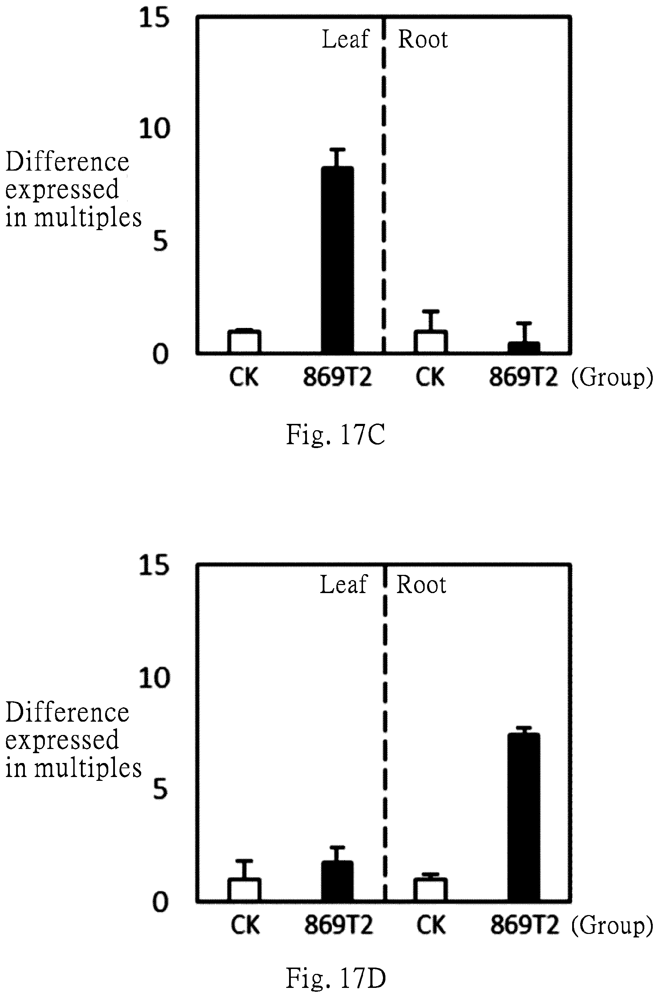

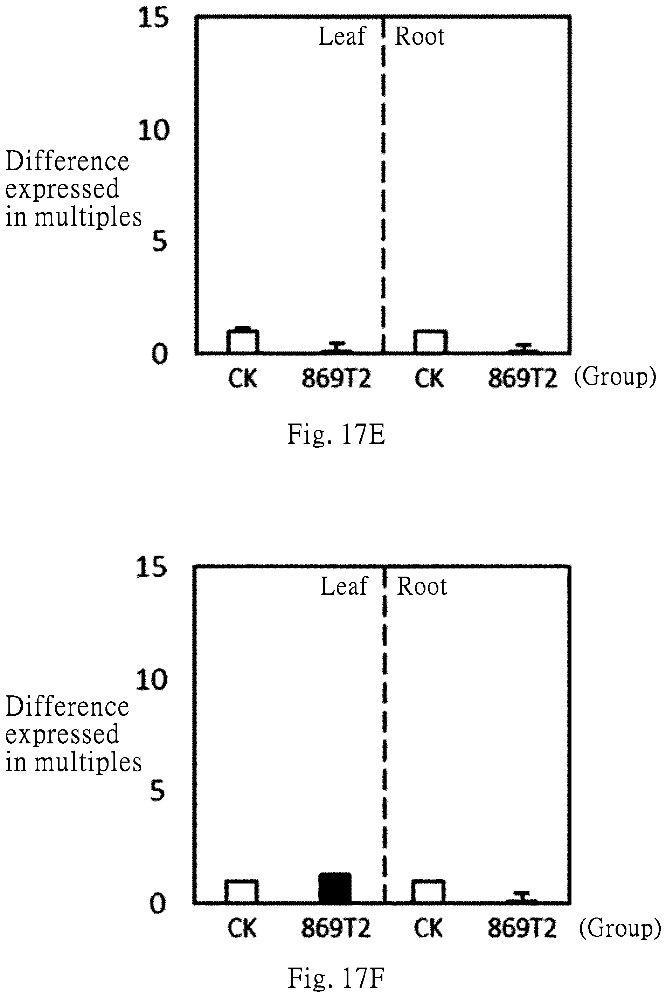

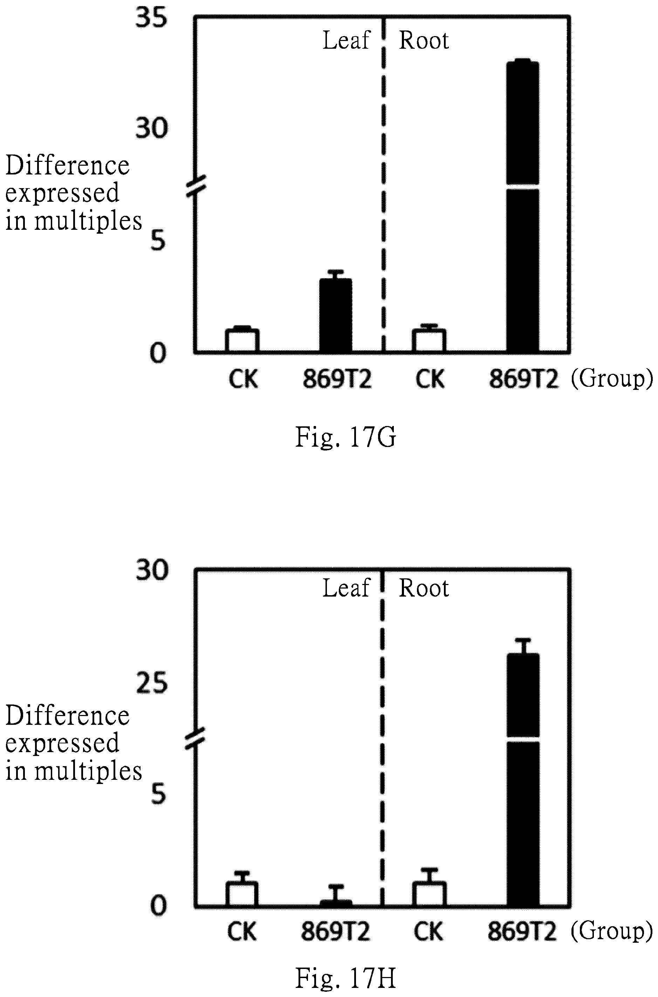

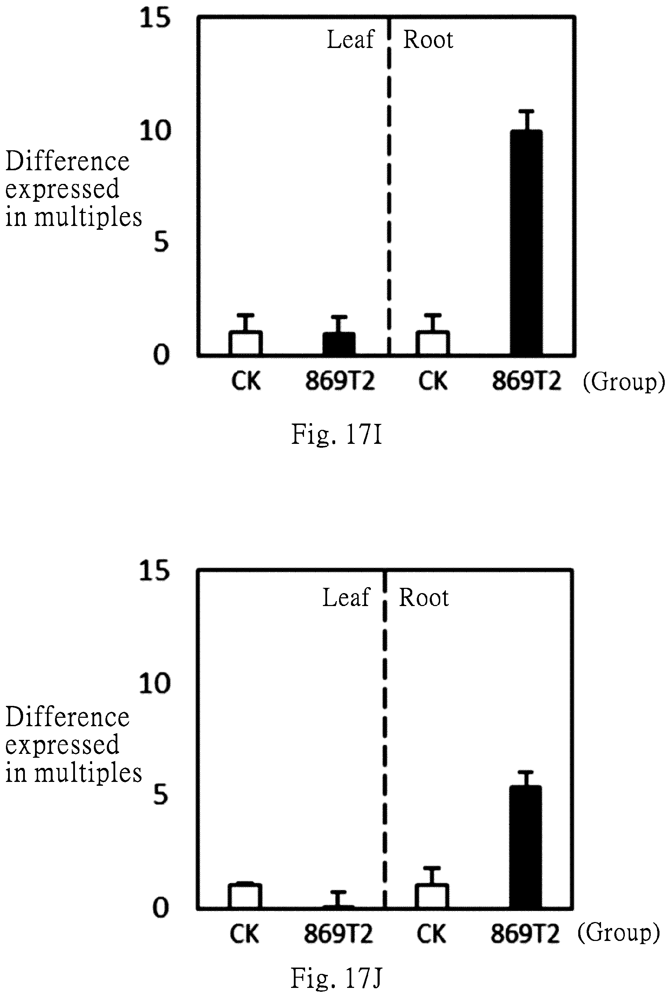

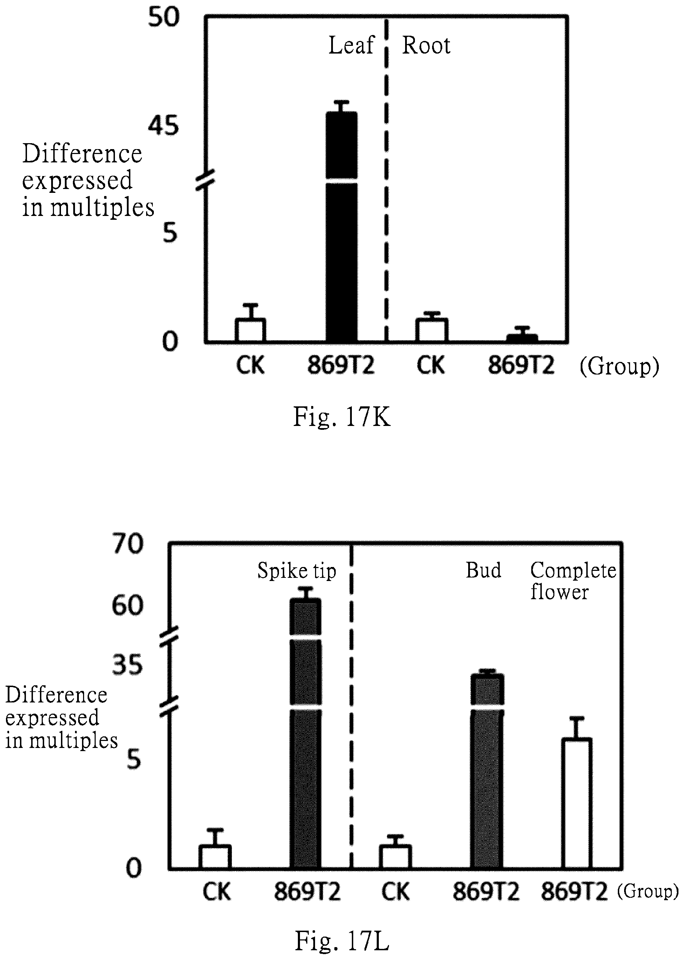

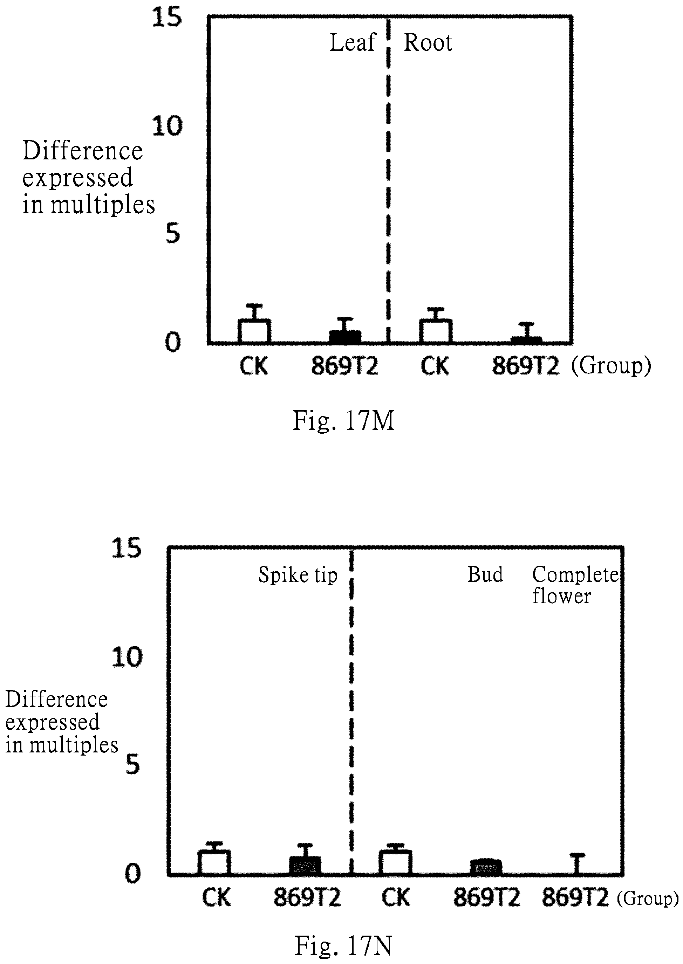

The relative expressions of the following genes in the leaves and roots of the *Phalaenopsis* orchids in the 869T2 group and the CK group were measured by quantitative real-time polymerase chain reaction (qRT-PCR) tests: *ATPases, RuBisCo small subunits, ALDH, Aux*/*IAA, ABCB1, ABCG2,* and *PDR5*; and so were the relative gene expressions of *FYF2* and *EDF* in the roots, leaves, buds, spike tips, and complete flowers of the *Phalaenopsis* orchids in the 869T2 groups and the CK group. The results are shown in FIG. 17.

As shown in FIG. 17A to FIG. 17J, the qRT-PCR test results indicate that the gene expressions of *ATPases, RuBisCo small subunits, ALDH, Aux*/*IAA, ABCB1, ABCG2,* and *PDR5* were consistent with the analysis result of the 869T2 group (gene transcription group) in example 5. It can also be known from the results in FIG. 17A to FIG. 17D that the *RuBisCo small subunits* in the leaves, the chloroplast *ATPase* gene and mitochondrial *ATPase* gene in the roots, and the *ALDH* gene in the roots showed a significant increase in expression, and this proves that the strain 869T2 disclosed in the present invention is indeed capable of stimulating photosynthesis and respiration of *Phalaenopsis* orchids and thereby affecting the carbon flow and energy production in *Phalaenopsis* orchids.

Moreover, it can be known from the results in FIG. 17E and FIG. 17F that *ACS7* and *ACS3,* which two genes encode 1-aminocyclopropane-1-carboxylate (ACC) synthase, showed a decrease in expression, and this proves that the strain 869T2 disclosed in the present invention has the activity to inhibit the substrate of ethylene biosynthesis and can therefore delay the withering of orchid flowers. It can also be known from the results in FIG. 17G that the *IAA1* gene in the orchid leaves and roots showed a significant increase in expression, and this proves that the strain 869T2 disclosed in the invention can affect the flowering periods and growth periods of orchids by regulating the auxin signaling pathway. It can be further known from the results in FIG. 17H to FIG. 17J that *ABCB1, ABCG2,* and *PDR5,* which three genes encode ATP-binding cassette (ABC) proteins, showed a significant increase in expression in the 869T2 group, and this proves that the strain 869T2 disclosed in the invention has the activity to participate in metabolite transport.

In addition, it can be known from the results in FIG. 17K to FIG. 17N that the *EDF* in all the plant tissues showed a decrease in expression, and that the *FYF2* in all the plant tissues except for the roots showed an increase in expression. These results prove that the strain 869T2 disclosed in the present invention can inhibit endogenous ethylene synthesis and can regulate ethylene signaling through a specific MADS-box family of transcription factors and thereby regulate the growth and flowering of orchids.

### Example 7: Analysis of the metabolite of strain 869T2

The metabolite of strain 869T2 was analyzed by ultra-high performance liquid chromatography-tandem mass spectrometry (UHPLC-MS/MS) and non-targeted metabolomic analysis, and the plant hormones and the related regulatory factors in the metabolite were identified. The analysis and identification results show that the metabolite produced by strain 869T2 during its growth contains the substances listed in Table 2.

It can be known from the results in Table 2 that the metabolite of the strain 869T2 disclosed in the present invention contains rhizobitoxin, which is a known *ACS* inhibitor. Therefore, administering the metabolite of the strain 869T2 disclosed in the invention or a composition containing the metabolite to a plant in the family *Orchidaceae* can reduce the expressions of the *ACS3* and *ACS7* genes in the plant, thereby inhibiting endogenous ethylene synthesis and ethylene signaling in the plant so that the flowering period can be extended, and postponed or suspended the flower senescence.

## Claims

1. A use of *Burkholderia seminalis* strain 869T2 or a metabolite thereof in preparing an ethylene synthesis inhibitor, wherein the *Burkholderia seminalis* strain 869T2 was deposited with Deutsche Sammlung von Mikroorganismen and Zellkulturen GmbH, Inhoffenstrasse 7B, D-38124 Braunschweig, Germany (DSMZ) on May 5, 2025.

2. The use of claim 1, wherein the metabolite of the *Burkholderia seminalis* strain 869T2 contains rhizobitoxin.

3. The use of claim 1, wherein the ethylene synthesis inhibitor is usable to regulate expression of flowering and ethylene synthesis-related genes in a plant in the family *Orchidaceae,* wherein the genes comprise *ACS7, ACS3, FT1 (FLOWERING LOCUS T1*)*, TFL1 (TERMINAL FLOWER1*), *LFY* (*LEAFY), EDF, FYF2,* and*IAA1.*

4. A method of regulating flowering of a plant in the family *Orchidaceae,* comprising: administering the ethylene synthesis inhibitor in any of claims 1 to 3 to the plant in the family *Orchidaceae* or to a cultivation medium of the plant in the family *Orchidaceae* in order to shorten a flower induction period, and extend a flowering period, of the plant in the family *Orchidaceae.*

5. The method of regulating flowering of a plant in the family *Orchidaceae* as claimed in claim 4, wherein the plant in the family *Orchidaceae* is in the genus *Phalaenopsis.*

6. The method of regulating flowering of a plant in the family *Orchidaceae* as claimed in claim 4, wherein the ethylene synthesis inhibitor is administered before the plant in the family *Orchidaceae* enters the flowering period.

7. The method of regulating flowering of a plant in the family *Orchidaceae* as claimed in claim 6, wherein the ethylene synthesis inhibitor is administered in stage 1 to stage 3 of a young plant period of the plant in the family *Orchidaceae.*

8. The method of regulating flowering of a plant in the family *Orchidaceae* as claimed in claim 7, wherein the ethylene synthesis inhibitor is administered at least once in each said stage of the plant in the family *Orchidaceae.*
